# FASCICULE DE BREVET EUROPEEN

(11) **EP 1 320 580 B1**
(45) Date de publication et mention de la délivrance du brevet: **03.06.2009**
(21) Numéro de dépôt: 01974388.9
(22) Date de dépôt: 20.09.2001
(51) Int. Cl.: C12N 1/28, A23C 9/127, A23C 19/032, C12N 1/20

(54) **ACTIVATEUR POUR FERMENT À BASE DE BACTÉRIES LACTIQUES ET PROCÉDÉ DE PRÉPARATION D'UN PRODUIT LACTÉ METTANT EN OEUVRE LEDIT ACTIVATEUR.**
AKTIVATOR FÜR EINE MILCHSÄUREBAKTERIELLE STARTERKULTUR, UND VERHFAHREN ZUR HERSTELLUNG EINES MILCHPRODUKTES UNTER BENÜTZUNG DIESES AKTIVATORS
ACTIVATOR FOR STARTER CULTURE OF LACTIC ACID BACTERIA AND METHOD FOR PREPARING A DAIRY PRODUCT USING THE SAME

(30) Priorité: 25.09.2000 FR 0012172; 23.02.2001 FR 0102492
(43) Date de publication de la demande: 25.06.2003
(73) Titulaire: DANISCO A/S, 1411 Copenhagen K. (DK)
(72) Inventeur: ZINDEL, Laurent, F-86100 CHATELLERAULT (FR); MORNET, Annie, F-86230 MONDION (FR); FONTAINE, Eloi, F-37000 TOURS (FR); GUILLAUD, Denis, F-38850 PALADRU (FR)
(74) Mandataire: Touati, Catherine
(86) Numéro de dépôt international: PCT/FR2001/002928
(87) Numéro de publication internationale: WO 2002/024870

(56) Documents cités:
- EP-A- 0 575 951
- WO-A-99/09838
- US-A- 4 020 185
- US-A- 4 402 986
- US-A- 4 851 347
- LELIEVELD H L M: "CONTINUOUS FERMENTATION IN YOGHURT MANUFACTURE" PROCESS BIOCHEMISTRY, XX, XX, vol. 11, no. 5, 1 juin 1976 (1976-06-01), pages 39-40, XP002031924
- GOMES ET AL.: "Use of small ruminants' milk supplemented with availabel nitrogen as growth media for Bifidobacterium lactis and Lactobacillus acidophilus" JOURNAL OF APPLIED MICROBIOLOGY, vol. 85, no. 5, novembre 1998 (1998-11), pages 839-848, XP001009572
- BANNIKOVA L A ET AL: "Starter for cows milk koumiss" INTERNATIONAL FOOD INFORMATION SERVICE (IFIS) FSTA,XX,XX, vol. 27, 1970, XP002136043

## Description

La présente invention se rapporte à un activateur pour un ferment à base de bactéries lactiques, à l'utilisation de cet activateur pour la préparation de produits lactés et au procédé de préparation d'un produit lacté caractérisé par la mise en oeuvre de cet activateur.

La fermentation du lait est généralement réalisée par ensemencement de celui-ci à l'aide d'une culture bactérienne couramment désignée sous le nom de starter, ferment ou levain. Ce ferment contient généralement des bactéries anaérobies ou microaérophiles appartenant au groupe des grams positifs qui fermentent les sucres en leurs acides respectifs. L'acide principalement produit est l'acide lactique à partir du lactose.

Généralement, ces ferments contiennent des organismes mésophiles ayant une température de croissance optimale comprise entre 25 et 35°C et/ou des organismes dits thermophiles ayant une température de croissance optimale entre 35 et 45°C.

Les organismes les plus utilisés et qui sont présents dans les ferments, sont ceux appartenant aux genres *Lactococcus, Streptococcus, Lactobacillus, Leuconostoc, Pediococcus, Bifidobacterium* et *Brevibacterium.*

Les organismes spécifiques appartenant au groupe des mésophiles comprennent les *Lactococcus lactis subsp. lactis, Lactococcus lactis subsp. cremoris, Lactococcus lactis subsp. lactis biovar. diacetylactis, Leuconostoc cremoris, Leuconostoc mesenteroides subsp mesenteroides, Leuconostoc mesenteroides subsp lactis, cette liste n'étant pas exhaustive.*

Les espèces bactériennes de type thermophile sont, entre autre, les *Streptococcus thermophilus, Lactobacillus casei, Lactobacillus paracasei, Lactobacillus helveticus, Lactobacillus delbrueckii subsp. Bulgaricus, Lactobacillus bulgaricus* et *Lactobacillus acidophilus.*

Ces ferments sont utilisés sous la forme de concentrés soit sous forme sèche, c'est-à-dire sous forme d'une poudre, par exemple lyophilisée ou atomisée, sous forme liquide, ou à l'état congelé.

Ces types de formulation ont pour double avantage de préserver la viabilité des cultures sur une large période et d'être tout particulièrement appropriés à l'ensemencement direct, selon lequel on introduit directement le ferment dans le lait de fabrication. Avantageusement, aucune mise en culture préliminaire ne s'avère nécessaire avant utilisation par opposition à l'ensemencement dit semi-direct.

Bien que la présente invention puisse également être appliquée efficacement à l'ensemencement semi-direct, elle s'avère tout particulièrement intéressante pour l'ensemencement dit direct pour la raison suivante : lorsque les bactéries sont introduites lors d'un ensemencement direct, c'est-à-dire sous la forme d'un concentrat sec, liquide ou congelé, elles ne sont pas immédiatement efficaces et elles nécessitent une remise en activité. La remise en activité de ce type de ferment nécessite un laps de temps d'adaptation correspondant d'une part au rétablissement de la bactérie conditionnée sous sa forme naturelle et d'autre part à la restitution de son activité métabolique. Plus précisément, cette période d'adaptation comprend successivement une première phase de réhydratation et une deuxième phase dite « de latence » correspondant plus particulièrement au « réveil » de l'activité métabolique des bactéries. C'est au cours de cette deuxième phase que s'effectuent la réparation cellulaire, l'adaptation du système enzymatique à son environnement biologique et l'initiation de la division cellulaire. Alors que la phase de réhydratation est quasiment immédiate, la phase de latence peut s'étendre jusqu'à 3 heures, et est bien entendu préjudiciable pour l'industriel en terme de rentabilité.

La technique de l'ensemencement direct offre des avantages déterminants : disponibilité immédiate des ferments sous un encombrement réduit, possibilité de réaliser des mélanges complexes d'espèces ou de souches différentes dans des proportions déterminées et constantes, régularité des performances accrue par rapport aux ferments traditionnels préparés sur les lieux d'utilisation, production réalisée dans des unités spécialisées où chaque étape du procédé est optimisée et contrôlée, qualité des ferments rigoureusement définie.

La présente invention a précisément pour objectif de proposer un moyen pour réduire significativement cette période de latence.

De manière inattendue, les inventeurs ont mis en évidence que la mise en contact d'un ferment à base de bactéries lactiques et de préférence dit direct, avec un activateur conforme à l'invention, préalablement à son introduction dans le milieu lacté à traiter, permettait de diminuer significativement ladite période de latence.

La présente invention a donc pour premier objet un activateur pour un ferment à base de bactéries lactiques.

Elle a pour second objet l'utilisation de cet activateur pour activer en milieu liquide un ferment à base de bactéries lactiques.

Un autre aspect de la présente invention concerne un ferment à base de bactéries lactiques ainsi activé.

Enfin, la présente invention a pour quatrième objet un procédé de préparation d'un produit lacté caractérisé par la mise en oeuvre de cet activateur ou d'un ferment activé selon l'invention.

Plus précisément, la présente invention vise un activateur pour un ferment à base de bactéries lactiques, caractérisé en ce qu'il comprend au moins :
- une substance azotée,
- un système tampon capable de maintenir le pH d'activité des bactéries lactiques auxquelles doit être associé ledit activateur à une valeur comprise entre 5 et 7,
et qui est exempt en sucre(s) ajouté(s) métabolisable(s) par lesdites bactéries lactiques.

L'activateur revendiqué est particulièrement intéressant en terme de stabilité et/ou de gain de productivité, d'un ferment à ensemencement direct sous forme liquide.

C'est ainsi qu'en raison de l'absence dans l'activateur, de sucre(s) métabolisable (s), on n'initie pas, lors de la mise en contact de cet activateur avec le ferment à activer, de production importante d'acide lactique qui serait préjudiciable à la stabilité des bactéries lactiques. Il s'ensuit une plus grande stabilité dans le temps du ferment activé.

En conséquence, l'utilisation conjointe de l'activateur avec un ferment à base de bactéries lactiques permet avantageusement de préserver et standardiser l'activité métabolique des bactéries activées sur une période de temps prolongée comparativement à celle observée avec un même ferment sous une forme non activée.

De plus, de manière tout à fait avantageuse, l'utilisation de l'activateur avec un ferment permet de retarder la multiplication cellulaire ou tout simplement de limiter la multiplication cellulaire, tout en permettant aux ferments de reprendre leur activité métabolique et en maintenant efficace le ferment activé selon l'invention. Ceci est illustré par l'exemple 3.

Un ferment activé selon l'invention est de manière avantageuse, efficace sur une période s'étendant jusqu'à 72 heures, plus particulièrement sur une période s'étendant jusqu'à 48 heures, préférentiellement sur une période s'étendant jusqu'à 24 heures.

C'est ainsi qu'un ferment à base d'un *Lactococcus lactis* activé selon l'invention est efficace sur une période s'étendant jusqu'à 72 heures alors qu'un même ferment, mais non activé, manifeste une perte d'activité significative au-delà de trois heures.

Par ailleurs, les inventeurs ont constaté que la présence de l'activateur était avantageuse en terme d'équilibre des populations microbiennes du système activé. Ceci est notamment illustré par l'exemple 3 figurant ci-après.

En ce qui concerne le gain de productivité, il est principalement lié à la réduction de la période dite « de latence ».

Plus précisément, au sens de la présente invention, on entend désigner par « période de latence », le délai s'écoulant entre le moment de l'introduction du ferment, activé ou non, dans un milieu lacté et le moment où l'activité métabolique des bactéries lactiques présentes dans ce ferment est vérifiée par une diminution significative du pH du milieu lacté due à la formation de l'acide lactique. Cette diminution du pH dite significative est en fait une valeur arbitraire, dépendante de l'appareillage de mesure retenu. Toutefois, à titre indicatif et dans le cas de l'appareillage mis en oeuvre dans les exemples illustrant l'invention, cette diminution du pH est évaluée à environ 0,08. De manière plus générale, on peut estimer que cette diminution significative est atteinte lorsque le pH du milieu lacté traité a diminué d'environ 5 % de sa valeur initiale.

Ce gain de productivité est particulièrement significatif pour des ferments à base de bactéries lactiques comprenant totalement ou tout au moins majoritairement des bactéries de type mésophile. Avantageusement, l'association d'un activateur à un ferment à base de bactéries lactiques de type mésophile réduit la période de latence d'environ 10 à 25% de sa valeur standard.

En conséquence et comme il ressort des exemples figurant ci-après, un ferment à base de bactéries lactiques sous une forme lyophilisée, mélangé préalablement à son introduction dans le lait avec un activateur selon l'invention, restitue beaucoup plus rapidement un pouvoir acidifiant dans le lait comparativement au ferment standard, c'est-à-dire sous une forme non activée.

Les substances azotées, présentes dans l'activateur revendiqué, sont ou dérivent de préférence de substances azotées de type peptides et acides aminés et/ou d'une ou plusieurs protéines, laitières ou non.

A titre représentatif des protéines convenant à l'invention, on peut notamment citer la β-lactoglobuline, l'albumine et l'alpha lactalbumine, les caséines et dérivés comme la caséine lactique, la caséine présure et les caséinates, la kappa caséine et la beta caséine.

Comme autres exemples de substances azotées, on peut notamment citer les extraits de levure et plus préférentiellement un extrait de levure *Saccharomyces cerevisiae,* qui peuvent être associés aux protéines citées précédemment.

Cette fraction en substances azotées constitue environ 50 à 90 % et de préférence 60 à 80 % en poids de l'activateur.

L'activateur selon l'invention ne contient pas de sucre(s) ajouté(s) ce qui signifie qu'il ne peut y avoir d'autres sources de sucre(s) ajouté(s) dans cet activateur que les substances azotées.

Il n'est en effet pas à exclure que ces substances azotées puissent contenir une certaine quantité de sucre(s) métabolisable(s), selon la source de substances azotées utilisées.

En ce qui concerne le milieu tampon, il a pour rôle principal de stabiliser le pH du ferment activé à une valeur proche entre 5 et 7 au cours de sa période de réactivation. Sa présence s'avère particulièrement avantageuse lorsqu'il est destiné à être associé à un ferment comprenant principalement des bactéries lactiques de type mésophiles et thermophiles.

A titre illustratif des mélanges tampons pouvant convenir à l'invention, on peut notamment citer ceux comprenant des sels de type sels de magnésium et de calcium ainsi que des sels de carbonates, phosphates, citrates.

Il s'agit préférentiellement d'un mélange de carbonates et plus préférentiellement d'un mélange de carbonate de calcium et de carbonate de magnésium.

Selon une variante de l'invention, sont également associés aux substances azotées et mélange tampon, des éléments nutritifs utiles au maintien de l'activité métabolique des bactéries lactiques.

Ces éléments nutritifs incluent généralement des vitamines.

De même, peuvent être présents dans l'activateur revendiqué des co-facteurs utiles pour activer la glycolyse. A titre représentatif de ces co-facteurs, on peut en particulier citer les sels minéraux Ca²⁺, Mg²⁺, Mn²⁺, Cu²⁺ et Zn²⁺. Ils sont généralement utilisés à raison de 0,1 à 2%.

On peut également envisager d'incorporer dans l'activateur des agents de texture de type hydrocolloïdes, comme les gommes xanthane, guar, etc.

Plus précisément, l'activateur selon l'invention est exempt en sucre(s) ajouté(s) métabolisable(s) par lesdites bactéries lactiques.

Encore plus précisément, l'activateur selon l'invention comprend au plus 15 % en poids en sucres métabolisables par lesdites bactéries lactiques, de préférence au plus 10 % des dits sucres, et plus particulièrement au plus 5 % des dits sucres. Il est entendu qu'il s'agit de sucre(s) non ajouté(s) au sens défini plus haut.

A titre illustratif des activateurs revendiqués, on peut plus particulièrement citer ceux comprenant au moins du caséinate de calcium, à raison de 20 à 40% en poids, et à titre de mélange tampon, un mélange de carbonates de calcium et de carbonates de magnésium. Sont également de préférence présents dans cet activateur des extraits de levure et du sulfate de manganèse.

L'activateur revendiqué peut être obtenu par simple mélange de ses composants et se présente généralement sous une forme sèche, généralement pulvérulente. Toutefois, on peut également envisager de le formuler sous une forme lyophilisée ou congelée.

L'activateur revendiqué peut aussi se présenter sous forme liquide.

Selon une variante préférée de l'invention, l'activateur revendiqué se présente sous une forme stérilisée et est mis en oeuvre en respectant cet aspect stérile.

La présente invention a pour second objet l'utilisation d'un activateur conforme à la présente invention pour activer un ferment à base de bactéries lactiques préalablement à ou lors de l'ensemencement d'un milieu lacté.

Le ratio ferment sur activateur est compris entre 10 % et 70 % en poids sec, de préférence de 20 % à 60 % en poids sec.

L'utilisation de cet activateur pour activer en milieu liquide un ferment à base de bactéries lactiques présente l'avantage d'un ensemencement en ligne, automatisable, continu ou discontinu et aseptique.

L'invention a également pour objet un ferment à base de bactéries lactiques activé, caractérisé en ce qu'il associe à au moins des bactéries lactiques, un activateur conforme à l'invention.

En l'espèce, l'activateur *revendiqué est utilisé en quantité telle que ces* composants, à savoir les substances azotées et mélange tampon, sont présents en quantités suffisantes pour que l'on observe une activation significative du ferment à base de bactéries lactiques.

A titre indicatif, il est utilisé en quantité telle que sa quantité en substances azotées est ajustée à raison d'environ 160 à 300 % en poids par rapport aux poids de bactéries lactiques présentes dans le ferment à activer, de préférence environ 160 % à 250 %.

L'activateur peut être mélangé au ferment soit préalablement ou au moment de son utilisation. Toutefois, selon un mode de réalisation privilégié, on procède préalablement à son utilisation, à sa réhydratation en présence d'un activateur conforme à la présente invention. Généralement, cette association est réalisée dans un milieu liquide, de préférence l'eau.

L'activateur est réhydraté de manière que la quantité d'activateur soit comprise entre 5 % et 20 % en poids de suspension aqueuse, de préférence comprise entre 7 % et 15%.

La réhydratation et l'activation consécutive peuvent être réalisées à une température comprise entre 10°C et 20°C et de préférence sous agitation, de manière à optimiser l'activation et l'homogénéisation dans le temps. Le ferment activé est ensuite utilisé tel quel pour l'ensemencement, de préférence direct, d'un milieu lacté.

Les bactéries lactiques susceptibles d'être associées à un activateur conforme à l'invention incluent toutes les bactéries usuellement mises en oeuvre pour la production de produits lactés.

A titre indicatif de bactéries lactiques, on peut citer les bactéries appartenant aux genres *Lactococcus, Streptococcus, Lactobacillus, Leuconostoc* et *Pediococcus.*

On considère également comme bactéries lactiques, les bactéries utilisées dans le domaine laitier appartenant aux genres *Bifidobacterium, Propionibacterium* et *Brevibacterium.*

Il peut également s'agir de microorganismes plus particulièrement utilisés pour l'affinage et notamment employés dans l'industrie fromagère. A titre représentatif de ce second type de microorganismes, on peut notamment citer les *Penicillium roqueforti, Penicillium candidum, Geotrichum candidum, Tourla kefir et Saccharomyces kefir* et *Kluyveryomyces lactis.*

La présente invention a pour quatrième objet un procédé de préparation d'un produit lacté comprenant : ,
(i) la mise en contact d'un ferment à base de bactéries lactiques avec un activateur conforme à la présente invention, de manière à obtenir un ferment dit activé,
(ii) l'ensemencement du milieu lacté à traiter, de préférence le lait, avec ledit ferment sous une forme activée, et
(iii) l'incubation dudit milieu lacté dans des conditions favorables à l'activité métabolique des bactéries lactiques, de manière à obtenir le produit lacté attendu.

Au sens de la présente invention, le ferment obtenu à l'issu de la première étape (i) est dit activé dans la mesure où comparativement à sa forme standard, c'est-à-dire non associée à un activateur selon l'invention, il manifeste une activité bactérienne améliorée. Cette amélioration se manifeste en termes de stabilité et de gain de productivité comme discuté précédemment.

Pour ce qui est de l'étape préliminaire (i), à savoir la mise en contact du ferment avec l'activateur revendiqué, elle est généralement effectuée dans un délai suffisant à l'obtention de la forme activée et au sein d'un milieu liquide. La suspension correspondante peut être obtenue par ajout d'un liquide, de préférence un milieu aqueux, au mélange des deux composants ou par dispersion consécutive des deux composants dans ledit liquide.

Comme précisé précédemment, l'activateur est utilisé en quantité telle que sa quantité en substances azotées est ajustée à raison d'environ 160 à 300 % en poids par rapport aux poids de bactéries lactiques, de préférence environ 160 % à 250 %.

La mise en oeuvre du procédé selon l'invention peut se faire grâce à un dispositif d'ensemencement.

Le dispositif d'ensemencement préféré, pour mettre en oeuvre le procédé selon l'invention, se présente sous la forme d'un réservoir scellé.

Le réservoir scellé peut se présenter sous la forme d'une poche fermée munie d'un système d'agitation interne et de moyens d'entrée et de sortie.

Un des moyens d'entrée permet l'arrivée du milieu aqueux dans le réservoir scellé afin de réaliser l'étape (i). Le milieu aqueux est préalablement stérilisé, de préférence il est filtré sur membrane d'au plus 0,45 µm, plus particulièrement au plus 0,22 µm. Il est à noter que l'on peut utiliser l'eau du robinet.

La température du milieu aqueux à son arrivée dans le réservoir scellé, est comprise entre 5°C et 15°C, de préférence comprise entre 8°C et 12°C.

Un des autres moyens d'entrée permet l'arrivée de gaz dans le réservoir scellé. L'arrivée de gaz va permettre de mettre en oeuvre le système d'agitation interne du réceptacle.

Le système d'agitation interne est constitué d'une poche interne perméable. Dans ce cas le réservoir scellé comprend une poche interne perméable et une poche externe fermée. L'agitation est réalisée par injection successive de gaz dans la poche interne perméable, qui permet le transfert de la suspension de la poche interne perméable à la poche externe fermée.

On utilise avantageusement un gaz qui n'intervient pas dans la respiration et / ou l'oxydation des microorganismes, des ferments et des bactéries.

Le gaz injecté est un gaz chimiquement et biologiquement inerte, de préférence on injecte de l'argon, plus particulièrement de l'azote ou du dioxyde de carbone.

Par gaz biologiquement inerte, on entend un gaz qui n'intervient pas dans la multiplication et la dégradation des microorganismes.

La pression de gaz dans le réservoir scellé, au cours de l'agitation, est inférieure à 5 bars, de préférence inférieure à 1 bar.

L'injection de gaz peut également se faire par intervalle régulier de temps. De préférence on injecte le gaz sous pression par intervalle de temps compris entre 0,5 minute et 60 minutes.

L'agitation permet la mise en suspension des ferments et de l'activateur dans le milieu aqueux.

Après agitation, la suspension de ferments et de l'activateur est maintenue en suspension par injection de gaz selon le même principe d'injection successive de gaz dans la poche interne.

La vidange du réservoir scellé se fait de manière aseptique par le moyen de sortie, ce qui permet de réaliser l'étape (ii) du procédé.

Cette vidange est réalisée par injection de gaz à l'intérieur du réservoir scellé, ou par transfert de la suspension aqueuse de ferments et d'activateur à l'aide d'une pompe ou par gravité.

L'ensemencement du milieu lacté à traiter avec ledit ferment sous une forme activée (étape (ii)) est réalisé à un débit compris entre 10 ml/min et 1000 ml/min, de préférence compris entre 100 ml/min et 500 ml/min.

La mise en oeuvre de l'étape (ii) selon l'invention se fait à une température comprise entre 5°C et 40°C, de préférence comprise entre 10°C et 15°C.

La mise en oeuvre de l'étape (ii) selon l'invention se fait sur une période s'étendant jusqu'à 72 heures, plus particulièrement sur une période s'étendant jusqu'à 48 heures, préférentiellement sur une période s'étendant jusqu'à 24 heures.

L'étape (ii) peut se réaliser selon plusieurs variantes.

Une première variante du procédé au niveau de l'étape (ii) consiste à ensemencer le milieu lacté à traiter en une seule fois avec ledit ferment sous une forme activée. Ceci est réalisé par vidange du ou des réservoir(s) en une seule fois. Il s'agit d'un ensemencement en lot (un seul réservoir) ou multi-lots (plusieurs réservoirs).

Une seconde variante du procédé au niveau de l'étape (ii) consiste à ensemencer le milieu lacté à traiter de manière continue avec ledit ferment sous une forme activée.

Une troisième variante du procédé au niveau de l'étape (ii) consiste à ensemencer le milieu lacté à traiter de manière discontinue avec ledit ferment sous une forme activée.

Par discontinue on entend un cycle d'ensemencement réalisé de la manière suivante : on ensemence le milieu lacté à traiter pendant un laps de temps, puis on arrête l'ensemencement, puis on recommence l'ensemencement, ceci pendant plusieurs cycles.

Dans le cadre de cette troisième variante, l'ensemencement du milieu lacté à traiter avec ledit ferment sous une forme activée (étape (ii)) est réalisé à un débit compris entre 10 ml/min et 1000 ml/min, de préférence compris entre 100 ml/min et 500 ml/min, fait par intervalle régulier ou irrégulier de temps compris entre 1 minute et 600 minutes.

Il est à noter que le réservoir scellé est de manière avantageuse fixé sur un poste mobile qui peut-être déplacé sur toutes les parties de la chaîne industrielle, avant ou après l'étape (i) du procédé selon l'invention.

Le type de réservoir préféré pour la mise en oeuvre du procédé selon l'invention est du type jetable et / ou stérile.

Ce réservoir est constitué de préférence d'une matière flexible comme par exemple le polypropylène, le polyester, le polyamide, la cellulose ou de tout autre matériel flexible compatible avec les produits alimentaires, de préférence il est en polyéthylène.

L'avantage de la mise en oeuvre du procédé selon l'invention grâce au dispositif d'ensemencement tel que décrit ci-dessus, est de réaliser un ensemencement direct, à température ambiante, stérile, standardisé, adaptable à chaque type de production et qui garantit la qualité bactériologique.

Un autre avantage de la mise en oeuvre du procédé selon l'invention grâce au dispositif d'ensemencement tel que décrit ci-dessus est de simplifier et fiabiliser l'étape d'ensemencement du ferment lactique.

La présente invention s'étend également aux différentes formes de conditionnement de l'activateur revendiqué.

On peut en effet formuler l'activateur revendiqué sous un conditionnement distinct de celui du ferment à base de bactéries lactiques auquel il est destiné à être associé ou, à l'inverse, envisager un conditionnement commun au sein duquel sont présents, de manière séparée ou non, l'activateur revendiqué et le ferment à base de bactéries lactiques.

Cette seconde variante de conditionnement peut par ailleurs être conçue de manière à ce qu'elle soit adaptée au mélange préalable du ferment et de l'activateur et donc à la préparation du ferment dit activé préliminairement à l'ensemencement d'un milieu lacté.

Les exemples figurant ci-après sont présentés à titre illustratif et non limitatif de la présente invention.

### Méthode

Les bactéries lactiques, seules ou en mélange, présentent une grande diversité de comportements. Dans le cas de la présente invention, l'activité acidifiante a été retenue à titre de critère de caractérisation.

L'acidification d'un milieu lacté se fait selon l'ordre chronologique suivant :
- inoculation d'un lait (pH voisin de 6,6),
- accroissement de la population de bactéries lactiques grâce à l'hydrolyse du lactose du lait,
- production d'acide lactique par les bactéries qui se traduit par une diminution du pH du milieu lacté,
- arrêt de la croissance des bactéries qui sont inhibées progressivement par l'acide lactique formé,
- poursuite de la production d'acide jusqu'à un pH de 4,5.

L'activité acidifiante a été appréciée dans les exemples ci-après à l'aide d'un système automatique de suivi et de caractérisation des ferments lactiques par acquisition de mesure de pH en temps réel, encore désignée ci-après sous l'appellation CINAC.

Le CINAC est composé:
* d'électrodes combinées en verre de type Ingold (24 voies de mesures de pH placées dans des erlens contenant le milieu ensemencé et 8 voies de mesures de température)
* d'un bain-marie régulé par un thermostat et dans lequel sont placés les erlenmeyers
* d'une carte électronique fournissant un signal analogique et une interface électronique convertissant ce dernier en numérique
* d'un micro-ordinateur PC muni du logiciel ONC assurant les fonctions suivantes :
   - configuration du système
   - acquisitions, traitements et stockages des données
   - étalonnage des sondes à pH7 et pH4
   - calcul des descripteurs cinétiques
   - représentations graphiques des données traitées
   - conversions des données pour l'utilisation de ces dernières sur d'autres logiciels
   - programmation de cycles thermiques pour réguler la température du bain-marie
   - compensation des températures pour corriger les variations de ces dernières sur le pH
      (cette correction se fait grâce à un régulateur PID : proportionnel-intégral-dérivé)
   - exécution de procédures de test des données d'étalonnage afin de détecte les dysfonctionnements liés aux sondes.

Le CINAC traite les données en fournissant les courbes des cinétiques d'acidification et les descripteurs de ces dernières.

Les courbes, décrivant les cinétiques, représentent les évolutions du pH et de la vitesse d'acidification (dpH/dt), en fonction du temps. Elles témoignent des différentes étapes de la croissance : phase de réadaptation, accélération, phase exponentielle, ralentissement, phase stationnaire.

Les descripteurs retenus dans les exemples pour caractériser les cinétiques d'acidification sont :
* Ta = temps de latence en min (temps au bout duquel le pH a varié de 0.08 upH en dessous de sa valeur initiale)
* Vm = vitesse maximale d'acidification en upH/min (vitesse prise au maximum de la valeur absolue de la dérivée dpH/dt=f(t))
* temps 5,20 = temps pour obtenir un pH de 5,20 en minutes.

A partir de l'ensemble de ces paramètres il est possible d'apprécier un gain ou une perte de productivité.

### EXEMPLE 1

### Préparation d'un ferment concentré réhydraté selon l'invention.

Préliminairement, on prépare l'activateur selon l'invention dans un flacon stérile de 1 I contenant un barreau magnétique double anneaux de 45 mm. Les différents composants de ce mélange sont présentés dans le tableau I ci-après :

**TABLEAU I**

| Produits | Quantité (g) |
|---|---|
| Protéines laitières | 30 |
| Extrait de *S. cerivisiae* | 35 |
| Carbonate de calcium | 10 |
| Carbonate de magnésium | 10 |
| Sulfate de manganèse | 5 |

Les fractions protéiques et minérales constituant ce mélange sont pasteurisées à 85°C pendant 30 minutes puis elles sont mélangées et l'ensemble est lyophilisé.

Dans les exemples ci-après, l'activateur ainsi obtenu est ensuite mélangé à 50g de ferment lyophilisé et 870g d'eau stérile. Le mélange sec est versé dans de l'eau sous agitation magnétique et la dissolution se fait en quelques minutes. On obtient ainsi 1 litre d'une solution qui contient 50g de lyophilisé.

La température de la réhydratation du mélange résultant, à savoir lyophilisat et activateur revendiqué est conduite selon un cycle thermique dit « hiver ». Ce cycle restitue la remontée en température d'un ensemble de 25 I qui commence à 15°C et s'achève à une température de 20°C qui est atteinte en 20 H environ.

### EXEMPLE 2

### Mesure de l'activité acidifiante du concentré liquide obtenu selon l'exemple 1.

L'activité du concentré bactérien est appréciée en fonction du temps de stockage. Elle est mesurée après 20 minutes (considéré comme le temps T0), 3 heures, 6 heures, 16 heures et 24 heures de stockage.

Les souches testées sont des souches à dominante mésophile. Il s'agit plus précisément des souches RA 024, RM 034 et MA 014 qui sont des ferments lactiques commercialisés par RHODIA FOOD S.A.S.

La souche RA 024 est un mélange *Lactococcus lactis subsp. lactis, Lactococcus lactis subsp. cremoris* et *Streptococcus salivarius subsp. thermophilus.*

La souche MA 014 est un mélange *Lactococcus lactis subsp. lactis* et *Lactococcus lactis subsp. cremoris.*

La souche RM 034 est un mélange *Lactococcus lactis subsp. lactis, Lactococcus lactis subsp. cremoris, Lactococcus lactis subsp. lactis biovar diacetylactis et Streptococcus thermophilus.*

Le support d'ensemencement utilisé est du lait ½ écrémé à 30°C.

En raison de la concentration, on procède à une dilution pour pouvoir ensemencer les tests d'acidification.

Une activité témoin est lancée pour chaque test réalisé qui met en oeuvre 1 g de lyophilisat dans 200 ml de lait.

Les témoins sont des ensemencements directs avec du ferment non activé dans le lait de fabrication.

En raison de la concentration des ferments utilisés, on procède à une dilution du produit. Ainsi, 1 g de ferment est dissous dans 200 ml de lait qui est utilisé pour la mesure d'activité.

Dans le cas des essais réhydratés, on procède également à une dilution.

L'ensemencement doit être réalisé immédiatement pour ne pas pénaliser l'activité du concentrat bactérien.

### Mesure de l'activité acidifiante au cours du temps.

Les résultats obtenus avec chacune des souches sont présentés en tableaux II, III et IV ci-après.

Les données figurant dans ces tableaux rendent compte des gains obtenus en termes de stabilité et de productivité avec les ferments activés selon l'invention comparativement à leur forme non activée respective.

**TABLEAU II**

| A 014 activé | Essai activé puis stocké | | | | | |
|---|---|---|---|---|---|---|
| Temps de stockage | 1 H | 2 H | 4 H | 6 H | 8 H | 24h |
| Temps en min. pour avoir un pH de 5,20 | 380 | 370 | 385 | 385 | 380 | 380 |

| MA 014 non activé témoin | | | | | | |
|---|---|---|---|---|---|---|
| Temps en min pour avoir un pH de 5,20 | 400 | 400 | 400 | 400 | 400 | 400 |
| Gain en temps technologique en minutes obtenu avec la forme activée | 20 | 30 | 15 | 15 | 20 | 20 |

**TABLEAU III**

| RA 024 activé | Essai activé puis stocké | | | | | | |
|---|---|---|---|---|---|---|---|
| Temps de stockage | 1 H | 2 H | 4 H | 6 H | 8 H | 12 H | 24h |
| Temps en min. pour avoir un pH de 5,20 | 395 | 395 | 390 | 390 | 390 | 390 | 395 |

| RA 024 non activé témoin | | | | | | | |
|---|---|---|---|---|---|---|---|
| Temps en min pour avoir un pH de 5,20 | 410 | 410 | 410 | 410 | 410 | 410 | 410 |
| Gain en temps technologique en minutes obtenu avec la forme activée | 15 | 15 | 20 | 20 | 20 | 20 | 15 |

**TABLEAU IV**

| RM 034 activé | Essai activé puis stocké | | | | | | |
|---|---|---|---|---|---|---|---|
| Temps de stockage | 1 H | 2 H | 4 H | 6 H | 8 H | 12 H | 24h |
| Temps en min. pour avoir un pH de 5,20 | 425 | 425 | 425 | 420 | 415 | 415 | 415 |

| RM 034 non activé témoin | | | | | | | |
|---|---|---|---|---|---|---|---|
| Temps en min pour avoir un pH de 5,20 | 445 | 445 | 445 | 445 | 445 | 445 | 445 |
| Gain en temps technologique en minutes obtenu avec la forme activée | 20 | 20 | 20 | 25 | 30 | 30 | 30 |

### EXEMPLE 3

### Stabilité des populations microbiennes en présence d'un activateur conforme à l'invention.

Dans cet essai, est évaluée la stabilisation des populations sur une durée de 24 heures au sein des ferments RA 021, RA 022, RA 024 et RA 026 réhydratés en présence de l'activateur préparé selon l'exemple 1.

RA 021, RA 022 et RA 026 sont des souches comprenant un mélange de bactéries mésophiles et thermophiles à l'image de celui identifié pour la souche RA 024 et sont commercialisées par RHODIA FOOD S.A.S.

Les conditions du mélange du ferment à base de bactéries lactiques considéré et de l'activateur sont identiques à celles présentées dans l'exemple 2.

Les résultats sont présentés dans le tableau V ci-après.

**TABLEAU V**

| Mélanges Commerciaux | Groupe de souches | Temps de stockage | | | |
|---|---|---|---|---|---|
| | | T0 | 4h00 | 8h00 | 24h00 |
| | | | | | |
| | mésophile | 3.10E+10 | 3.30E+10 | 3.50E+10 | 3.20E+10 |
| RA021 | thermophile | 5.10E+09 | 5.00E+09 | 5.40E+09 | 6.50E+09 |
| | | | | | |
| | mésophile | 3.10E+10 | 3.00E+10 | 3.00E+10 | 3.00E+10 |
| RA022 | thermophile | 3.20E+09 | 4.00E+09 | 4.40E+09 | 5.90E+09 |
| | | | | | |
| | mésophile | 3.20E+10 | 3.50E+10 | 3.00E+10 | 3.40E+10 |
| RA024 | thermophile | 4.90E+09. | 5.30E+09 | 5.80E+09 | 6.70E+09 |
| | | | | | |
| | mésophile | 2.40E+10 | 2.60E+10 | 2.50E+10 | 2.20E+10 |
| RA026 | thermophile | 3.70E+09 | 4.20E+09 | 4.00E+09 | 4.00E+09 |

De ces résultats il ressort le comportement avantageux de l'activateur vis-à-vis de la population bactérienne présente dans le ferment, et notamment la faible multiplication cellulaire.

## Revendications

1. Activateur pour un ferment à base de bactéries lactiques, **caractérisé en ce qu'**il comprend au moins :
- une substance azotée,
- un système tampon capable de maintenir le pH d'activité des bactéries lactiques auxquelles doit être associé ledit activateur à une valeur comprise entre 5 et 7,
et qui est exempt en sucre(s) ajouté(s) métabolisable(s) par lesdites bactéries lactiques.

2. Activateur selon la revendication 1, **caractérisé en ce que** la substance azotée est ou dérive d'une substance azotée de type peptide et acide aminé et/ou d'une ou plusieurs protéines laitières où non.

3. Activateur selon la revendication 2, **caractérisé en ce que** la substance azotée comprend une protéine choisie parmi la β-lactoglobuline, l'albumine et l'alpha lactalbumine, les caséines et dérivés comme la caséine lactique, la caséine présure et les caséinates, la kappa caséine, la beta caséine.

4. Activateur selon l'une des revendications 1 à 3 **caractérisé en ce que** la substance azotée comprend en outre un extrait de levure.

5. Activateur selon l'une des revendications 1 à 4, **caractérisé en ce que** la fraction en substance(s) azotée(s) constitue 50 à 90% et de préférence 60 à 80% en poids de l'activateur.

6. Activateur selon l'une des revendications précédentes, **caractérisé en ce que** le mélange tampon est un mélange de carbonates, et de préférence un mélange de carbonate de calcium et de carbonate de magnésium.

7. Activateur selon l'une des revendications précédentes, **caractérisé en ce qu'**il comprend en outre des éléments nutritifs nécessaires au maintien de l'activité métabolique des bactéries lactiques.

8. Utilisation d'un activateur tel que défini dans les revendications 1 à 7 pour l'activation d'un ferment à base de bactéries lactiques, préalablement à ou lors de l'ensemencement direct dans un milieu lacté.

9. Utilisation selon la revendication 8, **caractérisée en ce que** la mise en contact dudit activateur avec le ferment à base de bactéries lactiques est réalisée en milieu liquide.

10. Ferment à base de bactéries lactiques activé, **caractérisé en ce qu'**il associe à au moins des bactéries lactiques, un activateur selon l'une des revendications 1 à 7.

11. Ferment à base de bactéries lactiques selon la revendication 10, **caractérisé en ce que** les bactéries lactiques sont des bactéries à dominance mésophile.

12. Ferment à base de bactéries lactiques selon la revendication 10 ou 11, **caractérisé en ce que** les bactéries lactiques et l'activateur sont associés au sein d'un milieu liquide.

13. Ferment à base de bactéries lactiques selon l'une des revendications 10 à 12, **caractérisé en ce que** l'activateur est utilisé en quantité telle que sa quantité en substances azotées est ajustée à raison d'environ 160 à 300 % en poids par rapport aux poids de bactéries lactiques.

14. Procédé de préparation d'un produit lacté comprenant :
(i) la mise en contact d'un ferment comprenant au moins des bactéries lactiques avec un activateur selon l'une des revendications 1 à 7, de manière à obtenir le ferment sous une forme activée,
(ii) l'ensemencement du milieu lacté à traiter, de préférence le lait avec ledit ferment sous une forme activée, et
(iii) l'incubation dudit milieu lacté dans des conditions favorables à l'activité métabolique desdites bactéries lactiques de manière à obtenir le produit lacté attendu.

15. Procédé selon la revendication 14, **caractérisé en ce que** la mise en contact du ferment à base de bactéries lactiques avec ledit activateur est réalisée au sein d'un milieu liquide.

16. Procédé selon l'une des revendications 14 ou 15, **caractérisé en ce que** la mise en oeuvre du procédé peut se faire grâce à un dispositif d'ensemencement.

17. Procédé selon la revendication 16, **caractérisé en ce que** le dispositif - d'ensemencement se présente sous la forme d'un réservoir scellé.

18. Procédé selon la revendication 17, **caractérisé en ce que** le réservoir scellé peut se présenter sous la forme d'un résevoir jetable et / ou fixé sur un poste mobile.

19. Procédé selon l'une des revendications 17 ou 18, **caractérisé en ce que** le réservoir scellé peut se présenter sous la forme d'une poche munie d'un système d'agitation interne et de moyens d'entrée et de sortie.

20. Procédé selon l'une des revendications 17 ou 19, **caractérisé en ce que** un des moyens d'entrée permet l'arrivée du milieu aqueux dans le réservoir scellé afin de réaliser l'étape (i).

21. Procédé selon l'une des revendications 17 à 20, **caractérisé en ce que** la température du milieu aqueux à son arrivée dans le réservoir scellé, est comprise entre 5°C et 15°C, de préférence comprise entre 8°C et 12°C.

22. Procédé selon l'une des revendications 17 à 21, **caractérisé en ce que** un des autres moyens d'entrée permet l'arrivée de gaz le dans le réservoir scellé.

23. Procédé selon l'une des revendications 17 à 22, **caractérisé en ce que** le gaz injecté est un gaz chimiquement et biologiquement inerte, de préférence de l'argon, plus particulièrement de l'azote ou du dioxyde de carbone.

24. Procédé selon l'une des revendications 17 à 23, **caractérisé en ce que** la pression de gaz dans le réservoir scellé, est inférieure à 5 bars, de préférence inférieure à 1 bar.

25. Procédé selon l'une des revendications 17 à 24, **caractérisé en ce que** l'injection de gaz se fait par intervalle régulier de temps, de préférence compris entre 0,5 minute et 60 minutes.

26. Procédé selon l'une des revendications 14 à 25, **caractérisé en ce que** l'étape (ii) peut se réaliser selon plusieurs variantes, soit en lot, soit en multi-lots, soit en continu ou en discontinu.

27. Procédé selon l'une des revendications 14 à 26, **caractérisé en ce que** l'étape (ii) est réalisée à un débit compris entre 10 ml/min et 1000 ml/min, de préférence compris entre 100 ml/min et 500 ml/min.

28. Procédé selon l'une des revendications 14 à 27, **caractérisé en ce que** la mise en oeuvre de l'étape (ii) se fait à une température comprise entre 5°C et 40°C, de préférence comprise entre 10°C et 15°C.

29. Procédé selon l'une des revendications 14 à 28, **caractérisé en ce que** la mise en oeuvre de l'étape (ii) se fait sur une période s'étendant jusqu'à 72 heures, plus particulièrement sur une période s'étendant jusqu'à 48 heures, préférentiellement sur une période s'étendant jusqu'à 24 heures.

## Claims

1. An activator for a ferment based on lactic acid bacteria, **characterized in that** it comprises at least: a nitrogenous substance, a buffer system capable of maintaining the pH for activity of the lactic acid bacteria with which said activator has to be combined at a value between 5 and 7, and which is free of added sugar(s) which can be metabolized by said lactic acid bacteria.

2. The activator as claimed in claim 1, **characterized in that** the nitrogenous substance is or results from a nitrogenous substance of the peptide and amino acid type and/or from one or more dairy or non-dairy proteins.

3. The activator as claimed in claim 2, **characterized in that** the nitrogenous substance comprises a protein selected from beta-lactoglobulin, albumin and alpha-lactalbumin, caseins and derivatives such as lactic casein, rennet casein and caseinates, kappa-casein, beta-casein.

4. The activator as claimed in one of claims 1 to 3, **characterized in that** the nitrogenous substance comprises, in addition, a yeast extract.

5. The activator as claimed in one of claims 1 to 4, **characterized in that** the fraction of nitrogenous substance(s) constitutes 50 to 90%, and preferably 60 to by weight of the activator.

6. The activator as claimed in one of the preceding claims, **characterized in that** the buffer mixture is a mixture of carbonates, and preferably a mixture of calcium carbonate and of magnesium carbonate.

7. The activator as claimed in one of the preceding claims, **characterized in that** it comprises, in addition, nutritive elements necessary for maintaining the metabolic activity of the lactic acid bacteria.

8. The use of an activator as defined in claims 1 to 7 for the activation of a ferment based on lactic acid bacteria, prior to or during direct inoculation into a milk medium.

9. The use according to claim 8, **characterized in that** said activator is brought into contact with the ferment based on lactic acid bacteria in a liquid medium.

10. An activated ferment based on lactic acid bacteria, **characterized in that** it combines an activator as claimed in one of claims 1 to 7 with at least lactic acid bacteria.

11. The ferment based on lactic acid bacteria as claimed in claim 10, **characterized in that** the lactic acid bacteria are predominantly mesophilic bacteria.

12. The ferment based on lactic acid bacteria as claimed in claim 10 or 11, **characterized in that** the lactic acid bacteria and the activator are combined in a liquid medium.

13. The ferment based on lactic acid bacteria as claimed in one of claims 10 to 12, **characterized in that** the activator is used in a quantity such that its content of nitrogenous substances is adjusted in an amount of about 160 to 300% by weight relative to the weight of lactic acid bacteria.

14. A process for preparing a milk product comprising:
(i) bringing a ferment comprising at least lactic acid bacteria into contact with an activator as claimed in one of claims 1 to 7, so as to obtain the ferment in an activated form,
(ii) inoculating the milk medium to be treated, preferably milk, with said ferment in an activated form, and
(iii) incubating said milk medium under conditions favorable to the metabolic activity of said lactic acid bacteria so as to obtain the expected milk product.

15. The process as claimed in claim 14, **characterized in that** the ferment based on lactic acid bacteria is brought into contact with said activator in a liquid medium.

16. The process as claimed in either of claims 14 and 15, **characterized in that** the method may be carried out by means of an inoculation device.

17. The process as claimed in claim 16, **characterized in that** the inoculation device is provided in the form of a sealed reservoir.

18. The process as claimed in claim 17, **characterized in that** the sealed reservoir may be provided in the form of a disposable reservoir and/or attached to a mobile station.

19. The process as claimed in either of claims 17 and 18, **characterized in that** the sealed reservoir may be provided in the form of a pouch provided with an internal stirring system and inlet and outlet means.

20. The process as claimed in either of claims 17 and 19, **characterized in that** one of the inlet means allows the arrival of the aqueous medium in the sealed reservoir in order to carry out step (i).

21. The process as claimed in one of claims 17 to 20, **characterized in that** the temperature of the aqueous medium on its arrival in the sealed reservoir is between 5°C and 15°C, preferably between 8°C and 12°C.

22. The process as claimed in one of claims 17 to 21, **characterized in that** one of the other inlet means allows the arrival of gas into the sealed reservoir.

23. The process according to one of claims 17 to 22, **characterized in that** the injected gas is a chemically and biologically inert gas, preferably argon, more particularly nitrogen or carbon dioxide.

24. The process as claimed in one of claims 17 to 23, **characterized in that** the gas pressure in the sealed reservoir is less than 5 bar, preferably less than 1 bar.

25. The process as claimed in one of claims 17 to 24, **characterized in that** the injection of gas is carried out over a regular time interval preferably of between 0.5 minute and 60 minutes.

26. The process as claimed in one of claims 14 to 25, **characterized in that** step (ii) may be carried out according to several variants, either in batches, or in multi-batches, or continuously or batchwise.

27. The process as claimed in one of claims 14 to 26, **characterized in that** step (ii) is carried out at a flow rate of between 10 ml/min and 1000 ml/min, preferably of between 100 ml/min and 500 ml/min.

28. The process as claimed in one of claims 14 to 27, **characterized in that** step (ii) is carried out at a temperature of between 5°C and 40°C, preferably of between 10°C and 15°C.

29. The process as claimed in one of claims 14 to 28, **characterized in that** step (ii) is carried out over a period extending up to 72 hours, more particularly over a period extending up to 48 hours, preferably over a period extending up to 24 hours.

## Patentansprüche

1. Aktivator für eine Milchsäurebakterienkultur, **dadurch gekennzeichnet, dass** er mindestens eine stickstoffhaltige Verbindung und einen Puffer, der in der Lage ist, den pH-Wert der Milchsäurebakterien, zu dem der Aktivator zugegeben werden soll, auf einen Wert zwischen 5 und 7 zu halten, umfasst und der frei ist von hinzugefügte(m/n) Zucker(n), der/die durch die Milchsäurebakterien abbaubar ist/sind.

2. Aktivator gemäß Anspruch 1, **dadurch gekennzeichnet, dass** die stickstoffhaltige Verbindung eine stickstoffhaltige Verbindung vom Peptid- und Aminosäuretyp und/oder ein oder mehrere Milch- oder Nichtmilchprotein(e) oder ein Derivat davon ist.

3. Aktivator gemäß Anspruch 2, **dadurch gekennzeichnet, dass** die stickstoffhaltige Verbindung in Protein umfasst, das ausgewählt ist aus β-Lactoglobulin, Albumin und α-Lactalbumin, Caseinen und Derivaten, wie Milchcasein, Labcasein und Caseinaten, κ-Casein und β-Casein.

4. Aktivator gemäß einem der Ansprüche 1 bis 3, **dadurch gekennzeichnet, dass** die stickstoffhaltige Verbindung weiterhin ein Hefeextrakt umfasst.

5. Aktivator gemäß einem der Ansprüche 1 bis 4, **dadurch gekennzeichnet, dass** der Anteil der stickstoffhaltigen Verbindung(en) 50 bis 90 % und vorzugsweise 60 bis 80 %, bezogen auf das Gewicht des Aktivators, ist.

6. Aktivator gemäß einem der vorangehenden Ansprüche, **dadurch gekennzeichnet, dass** das Puffergemisch eine Mischung aus Carbonaten und vorzugsweise eine Calciumcarbonat- und Magnesiumcarbonatmischung ist.

7. Aktivator gemäß einem der vorangehenden Ansprüche, **dadurch gekennzeichnet, dass** er weiterhin Nährstoffe umfasst, die notwendig sind, um die Abbauaktivität der Milchsäurebakterien zu erhalten.

8. Verwendung eines in den Ansprüchen 1 bis 7 definierten Aktivators, zur Aktivierung einer Milchsäurekultur, vorab oder während der direkten Kultivierung in einem Milchmedium.

9. Verwendung gemäß Anspruch 8, **dadurch gekennzeichnet, dass** das Kontaktieren des Aktivators mit den Milchsäurekulturen in einem flüssigen Medium erfolgt.

10. Aktivierte Milchsäurekulturen, **dadurch gekennzeichnet, dass** mindestens den Milchsäurebakterien ein Aktivator gemäß einem der Ansprüche 1 bis 7 beigefügt ist.

11. Milchsäurekulturen gemäß Anspruch 10, **dadurch gekennzeichnet, dass** die Milchsäurebakterien mesophile Bakterien sind.

12. Milchsäurekulturen gemäß Anspruch 10 oder 11, **dadurch gekennzeichnet, dass** die Milchsäurebakterien und der Aktivator inmitten eines flüssigen Mediums zusammengefügt sind.

13. Milchsäurekulturen gemäß einem der Ansprüche 10 bis 12, **dadurch gekennzeichnet, dass** der Aktivator in einer solchen Menge verwendet wird, dass die Menge der stickstoffhaltigen Verbindung auf ungefähr 160 bis 300 Gew.%, bezogen auf das Gewicht der Milchsäurebakterien, eingestellt ist.

14. Verfahren zur Herstellung eines Milchprodukts, umfassend:
(i) Kontaktieren einer Kultur, die mindestens Milchsäurebakterien umfasst, mit einem Aktivator der Ansprüche 1 bis 7, so dass eine aktivierte Kultur erhalten wird,
(ii) Kultivieren eines zu behandelnden Milchmediums, vorzugsweise der Milch mit der besagten aktivierten Kultur und
(iii) Inkubieren des Milchmediums unter Bedingungen, die den Stoffwechsel der Milchsäurebakterien fördern, so dass das erwartete Milchprodukt erhalten wird.

15. Verfahren gemäß Anspruch 14, **dadurch gekennzeichnet, dass** das Kontaktieren der Milchsäurekultur mit dem Aktivator in einem flüssigen Medium durchgeführt wird.

16. Verfahren gemäß Anspruch 14 oder 15, **dadurch gekennzeichnet, dass** die Umsetzung des Verfahrens mit Hilfe einer Kultiviervorrichtung erfolgen kann.

17. Verfahren gemäß Anspruch 16, **dadurch gekennzeichnet, dass** die Kultiviervorrichtung in Form eines versiegelten Behälters vorliegt.

18. Verfahren gemäß Anspruch 17, **dadurch gekennzeichnet, dass** der versiegelte Behälter in Form eines Einwegbehälters vorliegt und/oder auf einem beweglichen Teil befestigt ist.

19. Verfahren gemäß Anspruch 17 oder 18, **dadurch gekennzeichnet, dass** der versiegelte Behälter in Form einer Tasche vorliegen kann, die mit einem internen Rührapparat und Beschickungs- und Entsorgungsmittel ausgestattet ist.

20. Verfahren gemäß Anspruch 17 oder 19, **dadurch gekennzeichnet, dass** die Beschickungsmittel den Einlauf des wässrigen Mediums in den versiegelten Behälter gestatten, um Schritt (i) durchzuführen.

21. Verfahren gemäß einem der Ansprüche 17 bis 20, **dadurch gekennzeichnet, dass** die Temperatur des wässrigen Mediums beim Einlaufen in den versiegelten Behälter zwischen 5 bis 15°C und vorzugsweise zwischen 8 und 12°C ist.

22. Verfahren gemäß einem der Ansprüche 17 bis 21, **dadurch gekennzeichnet, dass** die anderen Beschickungsmittel die Zuführung von Gas in den versiegelten Behälter gestatten.

23. Verfahren gemäß einem der Ansprüche 17 bis 22, **dadurch gekennzeichnet, dass** das zugeführte Gas chemisch und biologisch inert, vorzugsweise Argon und besonders bevorzugt Stickstoff oder Kohlendioxid ist.

24. Verfahren gemäß einem der Ansprüche 17 bis 23, **dadurch gekennzeichnet, dass** der Gasdruck in dem versiegelten Behälter geringer als 5 bar und vorzugsweise geringer als 1 bar ist.

25. Verfahren gemäß einem der Ansprüche 17 bis 24, **dadurch gekennzeichnet, dass** die Zuführung des Gases in regelmäßigen Zeitabständen vorzugsweise zwischen 0,5 und 60 min erfolgt.

26. Verfahren gemäß einem der Ansprüche 14 bis 25, **dadurch gekennzeichnet, dass** Schritt (ii) in mehreren Varianten durchgeführt wird, d.h. in einer Charge, in mehreren Chargen, kontinuierlich oder diskontinuierlich.

27. Verfahren gemäß einem der Ansprüche 14 bis 26, **dadurch gekennzeichnet, dass** Schritt (ii) mit einem Durchfluss zwischen 10 ml/min und 1000 ml/min, vorzugsweise zwischen 100 ml/min und 500 ml/min durchgeführt wird.

28. Verfahren gemäß einem der Ansprüche 14 bis 27, **dadurch gekennzeichnet, dass** die Umsetzung des Verfahrensschritts (ii) bei einer Temperatur zwischen 5 und 40°C und vorzugsweise zwischen 10 und 15°C durchgeführt wird.

29. Verfahren gemäß einem der Ansprüche 14 bis 28, **dadurch gekennzeichnet, dass** die Umsetzung des Verfahrensschritts (ii) über eine Zeitspanne von bis zu 72 Stunden, vorzugsweise über eine Zeitspanne von bis zu 48 Stunden und besonders bevorzugt über eine Zeitspanne von bis zu 24 Stunden durchgeführt wird.
